(19) **Europäisches Patentamt / European Patent Office / Office européen des brevets**

(11) **EP 2 467 061 B1**

(12) **FASCICULE DE BREVET EUROPEEN**

(45) Date de publication et mention de la délivrance du brevet:
**28.06.2017 Bulletin 2017/26**

(21) Numéro de dépôt: **09782007.0**

(22) Date de dépôt: **19.08.2009**

(51) Int Cl.:
***A61B 5/11*** *(2006.01)*

(86) Numéro de dépôt international:
**PCT/EP2009/060740**

(87) Numéro de publication internationale:
**WO 2011/020504 (24.02.2011 Gazette 2011/08)**

(54) **SYSTEME ET PROCEDE DE DETECTION DE CRISE D'EPILEPSIE D'UNE PERSONNE EPILEPTIQUE ALLONGEE**

SYSTEM UND VERFAHREN ZUR ERKENNUNG EINES EPILEPTISCHEN ANFALLS BEI EINER ZU EPILEPSIE NEIGENDEN PERSON

SYSTEM AND METHOD FOR DETECTING AN EPILEPTIC SEIZURE IN A PRONE EPILEPTIC PERSON

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(43) Date de publication de la demande:
**27.06.2012 Bulletin 2012/26**

(73) Titulaires:
- **Commissariat à l'Énergie Atomique et aux Énergies Alternatives 75015 Paris (FR)**
- **Movea 38000 Grenoble (FR)**

(72) Inventeurs:
- **JALLON, Pierre F-38100 Grenoble (FR)**
- **BONNET, Stéphane F-69003 Lyon (FR)**

(74) Mandataire: **Nguyen Van Yen, Christian Marks & Clerk France Conseils en Propriété Industrielle Immeuble Visium 22, Avenue Aristide Briand 94117 Arcueil Cedex (FR)**

(56) Documents cités:
EP-A1- 2 023 268    US-A1- 2009 062 696
US-B1- 6 212 510

- JIN HE ET AL: "Real-time Daily Activity Classification with Wireless Sensor Networks using Hidden Markov Model" ENGINEERING IN MEDICINE AND BIOLOGY SOCIETY, 2007. EMBS 2007. 29TH ANNUAL INTERNATIONAL CONFERENCE OF THE IEEE, IEEE, PISCATAWAY, NJ, USA, 1 août 2007 (2007-08-01), pages 3192-3195, XP031150165 ISBN: 978-1-4244-0787-3
- NIJSEN T M E ET AL: "Detection of Subtle Nocturnal Motor Activity From 3-D Accelerometry Recordings in Epilepsy Patients" IEEE TRANSACTIONS ON BIOMEDICAL ENGINEERING, IEEE SERVICE CENTER, PISCATAWAY, NJ, US LNKD-DOI:10.1109/TBME.2007.895114, vol. 54, no. 11, 1 novembre 2007 (2007-11-01), pages 2073-2081, XP011194590 ISSN: 0018-9294
- BECQ G ET AL: "Collection and Exploratory Analysis of Attitude Sensor Data in an Epilepsy Monitoring Unit" ENGINEERING IN MEDICINE AND BIOLOGY SOCIETY, 2007. EMBS 2007. 29TH ANNUAL INTERNATIONAL CONFERENCE OF THE IEEE, IEEE, PISCATAWAY, NJ, USA, 22 août 2007 (2007-08-22), pages 2775-2778, XP031336783 ISBN: 978-1-4244-0787-3

EP 2 467 061 B1

**Description**

La présente invention porte sur un système et un procédé de détection de crise d'épilepsie d'une personne épileptique allongée.

Les crises d'épilepsie sont dues à des dysfonctionnements du cerveau, qui peuvent se manifester de diverses manières. Cette maladie touche entre 0.5% et 1% de la population. 70% de ces malades peuvent contrôler leurs crises d'épilepsie en utilisant des médicaments antiépileptiques. Pour les autres 30% de malades, la chirurgie peut être envisagée pour éliminer la zone épileptique, ou, en d'autres termes, les régions du cerveau déclenchant ces crises, pour que le patient n'ait plus de crises.

Beaucoup de symptômes d'une crise d'épilepsie sont des symptômes moteur. Ces symptômes peuvent être enregistrés et analysés avec plusieurs technologies comme la vidéo ou les capteurs de mouvement pour caractériser une crise d'un patient ou pour réaliser une détection de crise pour des raisons de sécurité.

Il est connu des procédés de traitement vidéo pour quantifier les activités moteur d'un patient durant un crise. Des marqueurs sont ainsi placés sur le patient. L'avantage principal de ce type de procédé est que dans la plupart des chambres d'hôpital une caméra est déjà utilisée. Les problèmes liés à ces procédés proviennent du fait que le mouvement est difficile à analyser automatiquement à partir d'une image à deux dimensions, et que si le marqueur disparaît du champ de vision, des incertitudes apparaissent. En outre, ces types de procédés peuvent uniquement être réalisés dans une chambre dans laquelle une caméra est disponible. Une autre approche pour la caractérisation moteur de crises d'épilepsie est d'utiliser des capteurs inertiel/magnétique. Ces capteurs ont rendu possible d'extraire les informations pertinentes concernant des mouvements humains par le traitement de signaux multidimensionnels. Beaucoup d'applications ont ainsi été développées, à partir de tels capteurs à bas coût et non invasifs. Parmi celles-ci, l'analyse de démarche posture est sans doute la plus célèbre, par exemple décrite dans "A magnetometer-based approach for studying human movements,", de S. Bonnet et R. Heliot, IEEE Transactions on Biomedical Engineering, vol. 54, no. 7, 2007 qui propose un traitement basé sur un magnétomètre 3D pour évaluer en temps réel une inclinaison du corps pour détecter les mouvements comme un changement de position de assis à debout. La caractérisation de mouvements dus à des causes neurologiques a également été étudiée : l'exploitation d'un accéléromètre a été effectuée, par exemple, pour la maladie de Parkinson et la détection de tremblements de la main, tels que respectivement décrits dans les documents "The measuring set and signal processing method for the characterization of human hand tremor,", de A. Chwaleba, J. Jakubowski, and K. Kwiatos, CADSM, 2003, et "Triaxial accelerometry: a method for quantifying tremor and ataxia," de J. D. Frost, IEEE Transactions on Biomedical Engineering, vol. 25, no. 49, 1978. Concernant l'épilepsie, les documents, "The potential value of 3d accelerometry for detection of motor seizures in severe epilepsy," de T. Nijsen and al., Epilepsy and Behavior, vol. 7, 2005, et "Detection of subtle nocturnal motor activity from 3d accelerometry recordings in epilepsy patients," de T. Nijsen and al., IEEE Transactions on Biomedical Engineering, vol. 54, 2007, se concentrent sur la distinction entre des mouvements nocturnes et des mouvements de crise. Aussi, des capteurs sont attachés à un patient pour détecter une période avec des activités motrices. Une des hypothèses admise de ce système est que la personne ne lit pas ou ne va pas à la salle de bain lorsque le système est activé.

On peut également citer à titre d'exemple la demande de brevet US2009/062696, le brevet US6212510 et le document "Real-time Daily Activity Classification with Wireless Sensor Networks using Hidden Markov Model" de Jin He et al, IEEE Engineering in medicine and biology society, 2007, pp. 3192-3195.

De tels systèmes permettent de détecter de longues périodes d'activités motrices, et donc ne fonctionnent que dans des conditions strictes, et ont une très faible qualité de détection de crise d'épilepsie dans des conditions de vie courante.

Un but de l'invention est de pallier les problèmes précédemment cités, par exemple d'améliorer la précision de détection d'une crise d'épilepsie d'une personne allongée.

Selon un aspect de l'invention, il est proposé un système de détection d'une crise d'épilepsie d'une personne allongée comprenant :

- au moins un capteur de mouvement à au moins un axe de mesure muni de moyens de fixation pour lier solidairement ledit capteur de mouvement à ladite personne ;
- des premiers moyens de détermination d'une première probabilité d'au moins un premier diagramme états-transitions de l'activité nocturne d'une personne allongée par rapport à des informations représentatives des signaux de mesure du capteur de mouvement, ledit premier diagramme comprenant des probabilités prédéfinies de transitions orientées entre deux états différents ou identiques, les probabilités des états dudit premier diagramme étant prédéfinies ;
- des deuxièmes moyens de détermination d'une deuxième probabilité d'au moins un deuxième diagramme états-transitions de crise d'épilepsie par rapport à des informations représentatives des signaux de mesure du capteur de mouvement, ledit deuxième diagramme comprenant des probabilités prédéfinies de transitions orientées entre deux états différents ou identiques, les probabilités des états dudit deuxième diagramme étant prédéfinies ;
- des moyens d'association d'un état de ladite personne en fonction des probabilités des signaux de mesure du capteur de mouvement ;
- des premiers moyens de calcul des rapports de la première probabilité et de la deuxième probabilité ; et
- des moyens de détection d'une crise d'épilepsie lorsqu'au moins un desdits rapports calculés est inférieur à un seuil.

Un tel système permet de détecter avec une fiabilité améliorée une crise d'épilepsie d'une personne allongée.
Selon un mode de réalisation, au moins un desdits diagrammes états-transitions est adapté pour utiliser un modèle de Markov caché.
Un modèle de Markov caché est défini par deux processus aléatoires : un premier qui est appelé "état" dans la présente demande et qui n'est pas observé, ou, en d'autres termes qui est caché, et un second qui est l'observation dont la densité de probabilité à un instant donné dépend de la valeur de l'état au même instant.
En l'espèce, un modèle de Markov caché est défini par :

- un processus discret non observé noté l'état et qui peut prendre w valeurs , par exemple cinq valeurs (w=5) parmi les suivantes: une activité de repos (état 1), une activité de petite agitation (état 2), une activité de tremblements (état 3), une activité d'agitation (état 4) et une activité de forte agitation (état 5). Cette variable, ou état est une suite de Markov d'ordre 1, et est donc caractérisé par les probabilités de passage d'un état à un autre. Dans le cadre de ce mode de réalisation, un diagramme état-transitions est défini par :

    o L'ensemble des w états, w valant 5 dans l'exemple décrit
    o Les probabilités de passage d'un état à un autre, également appelées probabilités états-transistions :

$$\left\{a_{i,j} = P\big(Etat = i \big| Etat = j\big)\right\}_{i,\,j\in[1,\cdots,w]^2}$$

- un second processus observé du modèle de Markov caché est le signal multidimensionnel d'informations représentatives, ou, en d'autres termes de caractéristiques extraites du signal observé et dont la densité de probabilité dépend de l'état (le processus caché) à un instant donné. Notons $O(n)$ ce signal multidimensionnel à l'instant $n$ et $\{b_i(O(n)) = P(O(n)|Etat = i)\}_{i\in[1,\ldots,w]}$ les w probabilités associées à ce signal, en fonction de l'état caché sous-jacent.

On définit un modèle de Markov caché par le couple d'ensemble $Modele = \{\{a_{i,j}\}_{i,j}, \{b_i\}\}$. L'adéquation entre le signal observé et un modèle donné est évaluée par la probabilité suivante : $P(O(0),\cdots,O(N-1)|Modele)$
Cette probabilité peut être estimée avec les méthodes classiques décrites dans l'état de l'art : "An introduction to hidden Markov models" de L.R. Rabiner et B.H. Juang, IEEE ASSP Magazine, January 1986, ou dans le livre "Inference in Hidden Markov Models" de Cappé, Moulines et Ryden de Springer, de la série "Springer series in statisctics".
Plusieurs modèles ou diagrammes états-transitions sont définis, correspondant à différents mouvements d'intérêt, par exemple le premier diagramme états-transitions de mouvement nocturnes, et deux deuxièmes diagrammes états-transitions de mouvement de crise type tonique, et de crise type clonique.
Une crise de type tonique correspond à des muscles contractés puis décontractés rapidement montrant des tremblements, et une crise de type clonique correspond à une forte agitation avec des mouvements brusques et amples.
Pour une série d'observations donnée, les différentes probabilités $J_k = P(O(0),\cdots, O(N-1)|Modele_k)$ sont calculées. $Modele_k$ représente l'ensemble des paramètres décrivant le modèle numéro k. Une alarme peut être déclenchée si les probabilités associées à des modèles de crises sont plus grandes, au-delà d'un certain seuil, aux probabilités associées à des mouvements d'une autre nature. En d'autres termes, on détecte une crise d'épilepsie lorsqu'au moins un des rapports calculés est inférieur à un seuil, un rapport correspondant au ratio d'une première probabilité sur une deuxième probabilité.
Si l'ensemble des modèles associées à des mouvements de crises d'épilepsie est noté $C$ et l'ensemble des mouvements "normaux" est noté N , on détecte une crise d'épilepsie lorsque la condition suivante est vérifiée :

$\exists i \in C$ tel que $\forall j \in N,\ J_j/J_i < \lambda_{i,j}$
$\lambda_{i,j}$ étant un seuil ad-hoc.

Selon un mode de réalisation, le système comprend, en outre :

- un filtre pour sélectionner, pour chaque axe de mesure du capteur de mouvement, des hautes fréquences supérieures à un premier seuil, et des basses fréquences inférieures à un deuxième seuil inférieur ou égal audit premier seuil ;
- des deuxièmes moyens de calcul d'une première valeur égale à une combinaison linéaire des variations respectives, entre deux intervalles de temps successifs, selon chaque axe de mesure, desdites basses fréquences par intervalle de temps ;

- des troisièmes moyens de calcul d'une deuxième valeur égale à la moyenne des énergies, selon chaque axe de mesure, desdites hautes fréquences ;
- des troisièmes moyens de détermination de la probabilité de ladite première valeur définie par une loi Gaussienne normale centrée ; et
- des quatrièmes moyens de détermination de la probabilité de ladite deuxième valeur définie par une loi du Chi-2 de degré de liberté égal au nombre d'axes de mesure pris en compte du capteur de mouvement;

lesdits moyens d'association étant adaptés pour utiliser les probabilités desdites basses et hautes fréquences. En d'autres termes, les moyens d'association sont adaptés pour utiliser les probabilités relatives à la première et à le deuxième valeurs, respectivement définies par les troisièmes et quatrièmes moyens de détermination. De tels moyens d'association permettent d'attribuer, pour chaque état, une probabilité d'occurrence en fonction de la première valeur et de la seconde valeur respectivement établies par les deuxièmes et troisièmes moyens de calcul.

La précision de détection est ainsi améliorée à coût réduit. Le signal multidimensionnel $O(n)$ et les probabilités associées $\{b_i(O(n)) = P(O(n)|Etat = i)\}_{i \in [1,...,w]}$ sont définies comme suit, avec par exemple w=5. Le signal $O(n)$ de caractéristiques extraites du signal est de dimension 2. Sa première composante $x(n)$ est la première valeur. Sa seconde composante $y(n)$ est la deuxième valeur. Chaque nouvel échantillon correspond à une valeur calculée sur un intervalle de temps pouvant valoir 1 s, ce qui correspond à une fréquence d'échantillonnage de 1 Hz, mais d'une façon générale, la fréquence d'échantillonnage peut varier de 0.1 à 10 Hz, et de façon préférentielle de 0.5 Hz à 4 Hz. Les essais expérimentaux ont montré qu'une fréquence de 1 Hz était satisfaisante.

Pour un type de mouvement p donné, la probabilité d'observation est définie comme suit :

$$P\big(O(n)\big|Mouvement\_p\big) = P_{BF}^{(p)}(x(n)).P_{HF}^{(p)}\big(y(n)\big)$$

$P_{BF}^{(p)}\big(x(n)\big)$ correspond à la densité de probabilité relative à la première valeur, $x(n)$ correspond au mouvement p, p étant un entier naturel, compris par exemple entre 1 et 50, typiquement entre 3 et 30, et $P_{HF}^{(p)}\big(y(n)\big)$ correspond à la densité de probabilité relative à la seconde valeur $y(n)$ correspondant au mouvement p.

Dans un mode de réalisation, la densité de probabilité d'obtention d'un couple de valeurs pour la composante basses fréquences et la composante hautes fréquences étant égale au produit de la densité de probabilité d'obtention de la valeur pour la composante basses fréquences et de la densité de probabilité d'obtention de la valeur pour la composante hautes fréquences, lesdites densités de probabilité ($P_{BF}(X)$, $P_{HF}(X)$) sont définies par les expressions suivantes, pour chaque type de mouvement p :

$$\begin{cases} P_{BF}^{(p)}(x(n)) = \dfrac{1}{\sqrt{2\pi}\,\sigma_x^{(p)}} \cdot e^{-\frac{x(n)^2}{2\sigma_x^{(p)^2}}} \\[4mm] P_{HF}^{(p)}\big(y(n)\big) = \dfrac{1}{\sqrt{2^k}\,\sigma_y^{(p)k}\,\Gamma\!\left(\dfrac{k}{2}\right)} \, y(n)^{\frac{k}{2}-1} e^{-\frac{y(n)}{2\sigma_y^{(p)^2}}} \end{cases}$$

dans lesquelles :

- k représente le degré de liberté de la composante hautes fréquences (HF) égal au nombre d'axes de mesures pris en compte dudit capteur de mouvement (CM) ;
- $\sigma_x^{(p)}$ représente la variance de x, représentative d'un type de mouvement p ;
- $\sigma_y^{(p)}$ représente la moyenne des racines carrées des énergies des composantes hautes-fréquences des axes de mesure considérés, représentative d'un type de mouvement p ;
- n représente l'indice d'échantillon ; et
- $\Gamma$ est la fonction gamma vérifiant $\Gamma\!\left(\dfrac{1}{2}\right) = \sqrt{\pi}$, $\Gamma(1) = 1$ et $\Gamma(z + 1) = z\,\Gamma(z)$ pour z réel.

Ainsi, les densités de probabilité réelles des signaux observées sont approximées par des densités de probabilités globalement adaptées à la plupart des mouvements.

Le nombre de couple $\left(\sigma_x^{(p)}, \sigma_y^{(p)}\right)$ et les valeurs respectives de ces couples sont choisis pour décrire une quantité de mouvements suffisamment exhaustive. Les états, identifiés par l'indice i, sont alors décrits comme des mouvements plus ou moins probables avec la modélisation suivante :

$$b_i\left(O(n) = [x(n), y(n)]^T\right) = \sum_p \alpha_{i,p} P\left(O(n)\middle|Mouvement\_p\right)$$

$$= \sum_p \alpha_{i,p} \frac{1}{\sqrt{2\pi}\,\sigma_x^{(p)}} \cdot e^{-\frac{x(n)^2}{2\sigma_x^{(p)2}}} \times \frac{1}{\sqrt{2^k}\,\sigma_y^{(p)k}\,\Gamma\left(\frac{k}{2}\right)} y(n)^{\frac{k}{2}-1} e^{-\frac{y(n)}{2\sigma_y^{(p)2}}}$$

Les coefficients $\alpha_{i,p}$ vérifient la contrainte suivante :

$$\sum_p \alpha_{i,p} = 1.$$

Ainsi, les densités de probabilité associées aux états sont décrites avec une grande précision, et les signaux observés modélisés de manière assez fine. Ainsi lorsque la personne est à l'état repos, un soubresaut, qui ne dure pas longtemps, n'a pas une probabilité nulle. Si la personne est dans un état de tremblement, un soubresaut est aussi possible. Néanmoins pour cet état, ce mouvement élémentaire durera plus longtemps. Cette modélisation permet de prendre en compte de telles subtilités.

Par exemple, le nombre de couples $\left(\sigma_x^{(p)}, \sigma_y^{(p)}\right)$ vaut 18, permettant de décrire 18 mouvements.

Par exemple, lesdits 18 couples sont obtenus par combinaison des valeurs suivantes : $\sigma_x[0]=5.10^{-3}, \sigma_x[1]=1,8.10^{-2}, \sigma_x[2]=3,5.10^{-2}, \sigma_x[3]=5.,510^{-2}, \sigma_x[4]=8.10^{-2}, \sigma_x[5]=1.10^{-1}$, et $\sigma_y[0]=1.10^{-2}, \sigma_y[1]=3.10^{-2}, \sigma_y[2]=8.10^{-2}$.

Si , p est un index tel que $p = m + 6n,$ les couples $\left(\sigma_x^{(p)}, \sigma_y^{(p)}\right)$ sont définis comme suit :

$$\left(\sigma_x^{(p)}, \sigma_y^{(p)}\right) = \left(\sigma_x[m], \sigma_y[n]\right)$$

Dans un mode de réalisation, prenant en compte 5 états (w=5), lesdits coefficients $\alpha_{i,p}$ sont définis comme suit, variant également entre 0 et 17 :

| $\alpha_{i,p}$ | $i = 1$ (repos) | $i = 2$ (petite agitation) | $i = 3$ (tremblements) | $i = 4$ (agitation) | $i = 5$ (forte agitation) |
|---|---|---|---|---|---|
| $p = 0$ | 0.2564 | 0 | 0 | 0 | 0 |
| 1 | 0.0513 | 0.0526 | 0 | 0 | 0 |
| 2 | 0.02564 | 0 | 0.04 | 0 | 0 |
| 3 | 0.2564 | 0.1579 | 0.04 | 0 | 0 |
| 4 | 0.0513 | 0.2632 | 0.16 | 0 | 0 |
| 5 | 0 | 0.0526 | 0.20 | 0 | 0 |
| 6 | 0.2564 | 0.1579 | 0.04 | 0 | 0 |
| 7 | 0.0513 | 0.2632 | 0.16 | 0.0926 | 0 |
| 8 | 0 | 0.0526 | 0.20 | 0.0926 | 0 |
| 9 | 0.0256 | 0 | 0 | 0.0370 | 0 |
| 10 | 0 | 0 | 0 | 0.1852 | 0 |
| 11 | 0 | 0 | 0.16 | 0.1852 | 0 |

(suite)

| $\alpha_{i,p}$ | $i = 1$ (repos) | $i = 2$ (petite agitation) | $i = 3$ (tremblements) | $i = 4$ (agitation) | $i = 5$ (forte agitation) |
|---|---|---|---|---|---|
| 12 | 0.0256 | 0 | 0 | 0.037 | 0.0556 |
| 13 | 0 | 0 | 0 | 0.1852 | 0.0556 |
| 14 | 0 | 0 | 0 | 0.1852 | 0.0556 |
| 15 | 0 | 0 | 0 | 0 | 0.2778 |
| 16 | 0 | 0 | 0 | 0 | 0.2778 |
| 17 | 0 | 0 | 0 | 0 | 0.2778 |

Selon un mode de réalisation, le premier diagramme états-transitions des activités nocturnes générales est défini comme suit. $a_{i,j}^{n}$ représente la probabilité de passage de l'état i à l'état j, $P(Etat = i|Etat = j)$, pour le n$^{\text{ième}}$ diagramme états -transitions.

| $a_{i,j}^{(1)}$ | $i = 1$ | $i = 2$ | $i = 3$ | $i = 4$ | $i = 5$ |
|---|---|---|---|---|---|
| $j = 1$ | 0.9 | 0.025 | 0.025 | 0.025 | 0.025 |
| $j = 2$ | 0.025 | 0.9 | 0.025 | 0.025 | 0.025 |
| $j = 3$ | 0.025 | 0.025 | 0.9 | 0.025 | 0.025 |
| $j = 4$ | 0.025 | 0.025 | 0.025 | 0.9 | 0.025 |
| $j = 5$ | 0.025 | 0.025 | 0.025 | 0.025 | 0.9 |

Selon un mode de réalisation un deuxième diagramme états-transitions correspondant à une crise d'épilepsie avec des manifestations cloniques est défini par :

| $a_{i,j}^{(2)}$ | $i = 1$ | $i = 2$ | $i = 3$ | $i = 4$ | $i = 5$ |
|---|---|---|---|---|---|
| $j = 1$ | 0 | 0.3 | 0.7 | 0 | 0 |
| $j = 2$ | 0 | 0.9 | 0.1 | 0 | 0 |
| $j = 3$ | 0 | 0.1 | 0.9 | 0 | 0 |
| $j = 4$ | 0 | 0.3 | 0.7 | 0 | 0 |
| $j = 5$ | 0 | 0.3 | 0.7 | 0 | 0 |

Selon un mode de réalisation, un deuxième diagramme états-transitions de crise d'épilepsie avec des manifestations toniques est défini par :

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0.7 | 0 | 0.3 |
| $j = 2$ | 0 | 0 | 0.7 | 0 | 0.3 |
| $j = 3$ | 0 | 0 | 0.9 | 0 | 0.1 |
| $j = 4$ | 0 | 0 | 0.7 | 0 | 0.3 |
| $j = 5$ | 0 | 0 | 0.3 | 0 | 0.7 |

Dans un mode réalisation, lesdits deuxièmes moyens de détermination étant adaptés pour déterminer une deuxième probabilité d'un deuxième diagramme états-transitions de crise d'épilepsie avec des manifestations cloniques, et d'un deuxième diagramme états-transitions de crise d'épilepsie avec des manifestations toniques. Des manifestations toniques comprennent des tremblements, et des manifestations cloniques comprennent de l'agitation.

Selon un mode de réalisation, les états et les probabilités des états sont identiques pour le premier diagramme états-transitions et pour les deuxièmes diagrammes états-transitions.

La mise en oeuvre est ainsi simplifiée.

Ainsi, trois diagrammes états-transitions de signaux sont définis avec les 5 états (repos, petite agitation, tremblements, agitation, forte agitation). Un premier décrivant des activités nocturnes "générales", un second décrivant des manifestations motrices de crises d'épilepsies toniques (avec des tremblements) et un troisième décrivant des manifestations motrices de crises d'épilepsie cloniques (avec de l'agitation)

Dans un mode de réalisation, il n'y a qu'un seul premier diagramme états-transitions correspondant aux activités nocturnes générales est défini par :

| $a_{i,j}^{(1)}$ | $i = 1$ | $i = 2$ | $i = 3$ | $i = 4$ | $i = 5$ |
|---|---|---|---|---|---|
| $j = 1$ | 0.9 | 0.025 | 0.025 | 0.025 | 0.025 |
| $j = 2$ | 0.025 | 0.9 | 0.025 | 0.025 | 0.025 |
| $j = 3$ | 0.025 | 0.025 | 0.9 | 0.025 | 0.025 |
| $j = 4$ | 0.025 | 0.025 | 0.025 | 0.9 | 0.025 |
| $j = 5$ | 0.025 | 0.025 | 0.025 | 0.025 | 0.9 |

Selon un mode de réalisation, un deuxième diagramme états-transitions correspondant à une crise d'épilepsie avec des manifestations cloniques est défini par :

| $a_{i,j}^{(2)}$ | $i = 1$ | $i = 2$ | $i = 3$ | $i = 4$ | $i = 5$ |
|---|---|---|---|---|---|
| $j = 1$ | 0 | 0.3 | 0.7 | 0 | 0 |
| $j = 2$ | 0 | 0.9 | 0.1 | 0 | 0 |
| $j = 3$ | 0 | 0.1 | 0.9 | 0 | 0 |
| $j = 4$ | 0 | 0.3 | 0.7 | 0 | 0 |
| $j = 5$ | 0 | 0.3 | 0.7 | 0 | 0 |

Dans un mode de réalisation, un autre deuxième diagramme états-transitions de crise d'épilepsie avec des manifestations toniques est défini par :

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0.7 | 0 | 0.3 |
| $j = 2$ | 0 | 0 | 0.7 | 0 | 0.3 |
| $j = 3$ | 0 | 0 | 0.9 | 0 | 0.1 |
| $j = 4$ | 0 | 0 | 0.7 | 0 | 0.3 |
| $j = 5$ | 0 | 0 | 0.3 | 0 | 0.7 |

Aussi, les trois modèles sont définis avec les, les trois diagrammes états-transitions (le premier diagramme états-transitions des activités nocturnes générales et les deux deuxièmes diagrammes états-transitions de crise d'épilepsie avec respectivement des manifestations cloniques et toniques), les cinq états définis, le signal multidimensionnel $O(n)$ et les probabilités associées $\{b_i(O(n))=P(O(n)|Etat=i)\}_{i\in[1,\dots,5]}$.

La mise en oeuvre est ainsi simplifiée.

Pour une observation donnée, correspondant à un intervalle de temps de quelques secondes à quelques minutes par exemple de 45s, ce qui correspond à N signaux O(n) mesurés, indicés de O(0) à O(N-1), on calcule les trois probabilités suivantes :

$$J_1 = P\big(O(0), \cdots, O(N-1) \big| Modele_1\big)$$

$$J_2 = P\big(O(0), \cdots, O(N-1) \big| Modele_2\big)$$

$$J_3 = P\big(O(0), \cdots, O(N-1) \big| Modele_3\big)$$

et les deux ratios suivants :

$$\varphi_1 = \frac{J_1}{J_2} \text{ et } \varphi_2 = \frac{J_1}{J_3}$$

D'une façon générale, $J_i$ correspond à la probabilité des observations sachant le modèle i. $J_i$ sera proche de 0 si l'observation ne correspond pas au modèle i. Inversement, $J_i$ sera proche de 1 si l'observation correspond au modèle i.

Une alarme est déclenché si $\varphi_1 < \lambda_1$, ou $\varphi_2 < \lambda_2$ où $\lambda_1$ et $\lambda_2$ sont des seuils choisis de manière ad-hoc. Par exemple : $\lambda_1 = 1.10^{-2}$ et $\lambda_2 = 1.10^{-4}$.

Selon un mode de réalisation, le système comprend, en outre, des moyens d'alerte adaptés pour prévenir d'une détection d'une crise d'épilepsie.

Ces moyens d'alerte permettent d'alerter des personnes à proximité ou à distance, par exemple, par alerte sonore ou visuelle.

Selon un mode de réalisation, ledit capteur de mouvement comprend un accéléromètre, un magnétomètre, ou un gyromètre.

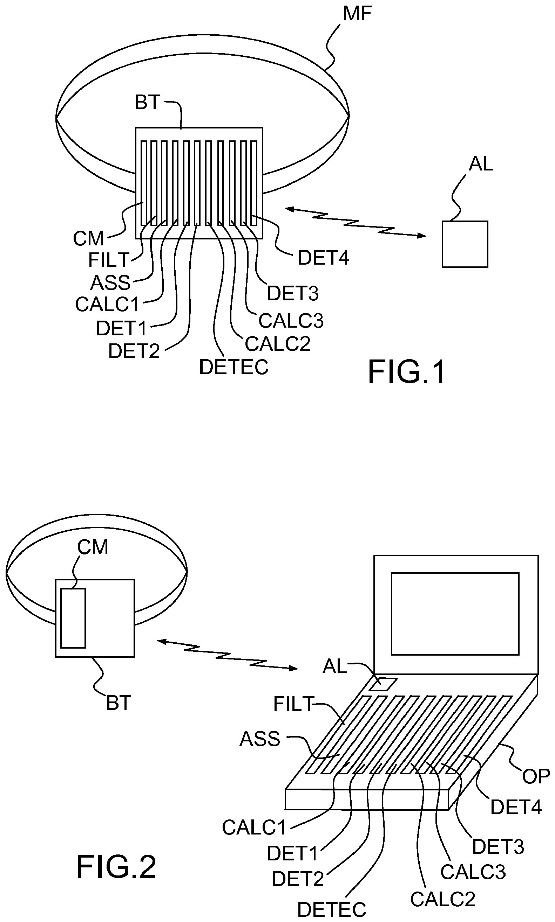

L'invention sera mieux comprise à l'étude de quelques modes de réalisation décrits à titre d'exemples nullement limitatifs et illustrés par les dessins annexés sur lesquels les figures 1 et 2 illustrent deux exemples de réalisation d'un système de détection d'une crise d'épilepsie d'une personne allongée, selon un aspect de l'invention.

La figure 1 illustre un système de détection d'une crise d'épilepsie d'une personne allongée, comprenant au moins un capteur de mouvement CM à au moins un axe de mesure muni d'éléments de fixation MF, comprenant par exemple un élément élastique, pour lier solidairement un boîtier BT comprenant le capteur de mouvement CM à ladite personne. Le capteur de mouvement CM peut être, un accéléromètre, un magnétomètre, ou un gyromètre, à un, deux, ou trois axes de mesure. Bien entendu, le système peut comprendre une pluralité de capteurs de mouvement CM.

Le système comprend un module d'association ASS d'un état de ladite personne en fonction des probabilités des signaux de mesure du capteur de mouvement CM, et un premier module de détermination DET1 d'une première probabilité d'un premier diagramme états-transitions de l'activité nocturne d'une personne allongée par rapport à des informations représentatives des signaux de mesure du capteur de mouvement CM. Le premier diagramme comprend des probabilités prédéfinies de transitions orientées entre deux états différents ou identiques, les probabilités des états dudit premier diagramme étant prédéfinies.

Le système comprend également un deuxième module de détermination DET2 d'une deuxième probabilité d'au moins un deuxième diagramme états-transitions de crise d'épilepsie par rapport à des informations représentatives des signaux de mesure du capteur de mouvement CM. Un deuxième diagramme comprend des probabilités prédéfinies de transitions orientées entre deux états différents ou identiques, les probabilités des états dudit deuxième diagramme étant prédéfinies.

Le système comprend, en outre, un premier module de calcul CALC1 des rapports de la première probabilité et de la deuxième probabilité, et un module de détection DETEC d'une crise d'épilepsie lorsqu'au moins un desdits rapports calculés est inférieur à un seuil.

Dans la suite de la description, on considère, par exemple, que le deuxième module de détermination DET2 met en oeuvre un deuxième diagramme états-transitions de crise d'épilepsie avec des manifestations cloniques, et un deuxième diagramme états-transitions de crise d'épilepsie avec des manifestations toniques.

Le système peut comprendre un module d'alerte ou d'alarme AL permettant de prévenir d'une détection d'une crise d'épilepsie, à proximité ou à distance, des personnes pouvant intervenir.

En variante, comme illustré sur la figure 2, de nombreux éléments peuvent être, non pas compris dans le boîtier BT, comme illustré sur la figure 1, mais compris, par exemple, dans un ordinateur portable OP.

Le premier diagramme états-transitions de l'activité nocturne d'une personne allongée, peut avoir pour probabilités de passages entre deux états desdits cinq états :

| $a_{i,j}^{(1)}$ | $i = 1$ | $i = 2$ | $i = 3$ | $i = 4$ | $i = 5$ |
|---|---|---|---|---|---|
| $j = 1$ | 0.9 | 0.025 | 0.025 | 0.025 | 0.025 |
| $j = 2$ | 0.025 | 0.9 | 0.025 | 0.025 | 0.025 |
| $j = 3$ | 0.025 | 0.025 | 0.9 | 0.025 | 0.025 |
| $j = 4$ | 0.025 | 0.025 | 0.025 | 0.9 | 0.025 |
| $j = 5$ | 0.025 | 0.025 | 0.025 | 0.025 | 0.9 |

Un premier exemple de deuxième diagramme états-transitions de crise d'épilepsie avec des manifestations cloniques, peut être défini par les probabilités de passages suivantes :

| $a_{i,j}^{(2)}$ | $i = 1$ | $i = 2$ | $i = 3$ | $i = 4$ | $i = 5$ |
|---|---|---|---|---|---|
| $j = 1$ | 0 | 0.3 | 0.7 | 0 | 0 |
| $j = 2$ | 0 | 0.9 | 0.1 | 0 | 0 |
| $j = 3$ | 0 | 0.1 | 0.9 | 0 | 0 |
| $j = 4$ | 0 | 0.3 | 0.7 | 0 | 0 |
| $j = 5$ | 0 | 0.3 | 0.7 | 0 | 0 |

Un deuxième exemple de deuxième diagramme états-transitions de crise d'épilepsie avec des manifestations toniques, peut être défini par les probabilités de passages suivantes :

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0.7 | 0 | 0.3 |
| $j = 2$ | 0 | 0 | 0.7 | 0 | 0.3 |
| $j = 3$ | 0 | 0 | 0.9 | 0 | 0.1 |
| $j = 4$ | 0 | 0 | 0.7 | 0 | 0.3 |
| $j = 5$ | 0 | 0 | 0.3 | 0 | 0.7 |

A partir des signaux de mesure du capteur de mouvement CM, le module d'association ASS détermine l'état de ladite personne parmi l'ensemble des états. Le premier module de détermination DET1 détermine une première probabilité du premier diagramme états-transitions par rapport à des informations représentatives des signaux de mesure du capteur de mouvement CM.

En outre, le deuxième module de détermination DET2 détermine une deuxième probabilité du deuxième diagramme états-transitions de crise d'épilepsie avec des manifestations clonique par rapport à des informations représentatives des signaux de mesure du capteur de mouvement CM, et détermine une deuxième probabilité du deuxième diagramme états-transitions de crise d'épilepsie avec des manifestations toniques par rapport aux signaux de mesure du capteur de mouvement CM.

Le premier module de calcul CALC1 estime le rapport $\varphi_1$ de la première probabilité du premier diagramme états-transitions et de la deuxième probabilité du deuxième diagramme états-transitions de crise d'épilepsie avec des manifestations cloniques, et le rapport $\varphi_2$ de la première probabilité du premier diagramme états-transitions et de la deuxième probabilité du deuxième diagramme états-transitions de crise d'épilepsie avec des manifestations toniques.

Le module de détection DETEC détecte une crise d'épilepsie lorsqu'au moins un des deux rapports calculés par le premier module de calcul CALC1 est inférieur à un seuil respectif $\lambda_1$ ou $\lambda_2$.

Dans la suite de la description, les exemples décrits utilisent les composantes basses fréquences BF et hautes fréquences HF, et les capteurs de mouvements CM sont au nombre de deux et sont deux accéléromètres triaxiaux, dont un premier est fixé à un poignet de l'utilisateur, et le deuxième est fixé à l'autre poignet de l'utilisateur ou à sa poitrine. Un ordinateur OP, tel qu'illustré par exemple sur la figure 2, reçoit et enregistre les données, détecte une crise d'épilepsie et déclenche une alerte en cas de détection de crise d'épilepsie. En outre, les diagrammes états-transitions décrits sont adaptés pour utiliser respectivement un modèle de Markov caché.

Au moins un desdits diagrammes états-transitions peut être adapté pour utiliser un modèle de Markov caché. En outre, le système peut comprendre un filtre FILT pour sélectionner, pour chaque axe de mesure du capteur de mouvement CM, des hautes fréquences HF supérieures à un premier seuil S1, et des basses fréquences BF inférieures à un deuxième seuil S2 inférieur ou égal audit premier seuil S1. Le système peut également comprendre un deuxième module de calcul CALC2 d'une première valeur x égale à une combinaison linéaire des variations respectives, entre deux intervalles de temps successifs, selon chaque axe de mesure, des basses fréquences BF par intervalle de temps n, et comprendre un troisième module de calcul CALC3 d'une deuxième valeur y égale à la moyenne des énergies, selon chaque axe de mesure, des hautes fréquences HF. En outre, le système peut comprendre un troisième module de détermination DET3 de la probabilité PBF(x) de ladite première valeur x définie par une loi Gaussienne normale centrée, et un quatrième module de détermination DET4 de la probabilité PHF(x) de ladite deuxième valeur y définie par une loi du Chi-2 de degré de liberté égal au nombre d'axes de mesure pris en compte du capteur de mouvement CM. Les moyens d'association ASS sont adaptés pour utiliser les probabilités des basses et hautes fréquences BF et HF.

La densité de probabilité P(x(n),y(n)) d'obtention d'un couple de valeurs (x(n), y(n)) pour la composante basses fréquences BF et la composante hautes fréquences HF étant égale au produit de la densité de probabilité $P_{BF}(x)$ d'obtention de la valeur x(n) pour la composante basses fréquences BF et de la densité de probabilité $P_{HF}(x)$ d'obtention de la valeur y(n) pour la composante hautes fréquences HF, les densités de probabilité $P_{BF}(x)$, $P_{HF}(x)$ étant définies par les expressions suivantes pour chaque type de mouvement p :

$$\begin{cases} P_{BF}^{(p)}(x(n)) = \dfrac{1}{\sqrt{2\pi}\ \sigma_x^{(p)}} \cdot e^{\frac{x(n)^2}{2\sigma_x^{(p)^2}}} \\[4mm] P_{HF}^{(p)}\big(y(n)\big) = \dfrac{1}{\sqrt{2^k}\ \sigma_y^{(p)k}\ \Gamma\left(\dfrac{k}{2}\right)}\ y(n)^{\frac{k}{2}-1}\ e^{\frac{y(n)}{2\sigma_y^{(p)^2}}} \end{cases}$$

dans lesquelles :

- k représente le degré de liberté de la composante hautes fréquences (HF) égal au nombre d'axes de mesures pris en compte dudit capteur de mouvement (CM) ;

- $\sigma_x^{(p)}$ représente la variance de x, représentative d'un type de mouvement p ;

- $\sigma_y^{(p)}$ représente la moyenne des racines carrées des énergies des composantes hautes-fréquences des axes de mesure considérés, représentative d'un type de mouvement p ;

- n représente l'indice d'échantillon ; et

- $\Gamma$ est la fonction gamma vérifiant $\Gamma\left(\dfrac{1}{2}\right) = \sqrt{\pi}$ , $\Gamma(1) = 1$ et $\Gamma(z + 1) = z\ \Gamma(z)$ pour z réel.

La présente invention permet ainsi, à coût réduit d'améliorer sensiblement la détection d'une crise d'épilepsie d'une personne allongée.

**Revendications**

**1.** Système de détection d'une crise d'épilepsie d'une personne allongée **caractérisé en ce qu'**il comprend :

- au moins un capteur de mouvement (CM) à au moins un axe de mesure muni de moyens de fixation (MF) pour lier solidairement ledit capteur de mouvement (CM) à ladite personne ;

- des premiers moyens de détermination (DET1) d'une première probabilité d'au moins un premier diagramme états-transitions de l'activité nocturne d'une personne allongée par rapport à des informations représentatives des signaux de mesure du capteur de mouvement (CM), ledit premier diagramme comprenant des probabilités prédéfinies de transitions orientées entre deux états différents ou identiques, les probabilités des états dudit premier diagramme étant prédéfinies ;

- des deuxièmes moyens de détermination (DET2) d'une deuxième probabilité d'au moins un deuxième diagramme états-transitions de crise d'épilepsie par rapport à des informations représentatives des signaux de mesure du capteur de mouvement (CM), un deuxième diagramme comprenant des probabilités prédéfinies de transitions orientées entre deux états différents ou identiques, les probabilités des états dudit deuxième diagramme étant prédéfinies ;

- des moyens d'association (ASS) d'un état de ladite personne en fonction des probabilités des signaux de mesure du capteur de mouvement (CM) ;

- des premiers moyens de calcul (CALC1) des rapports ($\varphi_1$, $\varphi_1$) de la première probabilité et de la deuxième probabilité ; et

- des moyens de détection (DETEC) d'une crise d'épilepsie lorsqu'au moins un desdits rapports calculés ($\varphi_1$, $\varphi_1$) est inférieur à un seuil ($\lambda_1$, $\lambda_2$).

2. Système selon la revendication 1, dans lequel au moins un desdits diagrammes états-transitions est adapté pour utiliser un modèle de Markov caché.

3. Système selon la revendication 2, comprenant, en outre :

- un filtre (FILT) pour sélectionner, pour chaque axe de mesure du capteur de mouvement (CM), des hautes fréquences (HF) supérieures à un premier seuil (S1), et des basses fréquences (BF) inférieures à un deuxième seuil (S2) inférieur ou égal audit premier seuil (S1) ;

- des deuxièmes moyens de calcul (CALC2) d'une première valeur (x) égale à une combinaison linéaire des variations respectives, entre deux intervalles de temps successifs, selon chaque axe de mesure, desdites basses fréquences (BF) par intervalle de temps (n) ;

- des troisièmes moyens de calcul (CALC3) d'une deuxième valeur (y) égale à la moyenne des énergies, selon chaque axe de mesure, desdites hautes fréquences (HF) ;

- des troisièmes moyens de détermination (DET3) de la probabilité ($P_{BF}(x)$) de ladite première valeur (x) définie par une loi Gaussienne normale centrée ; et

- des quatrièmes moyens de détermination (DET4) de la probabilité ($P_{HF}(x)$) de ladite deuxième valeur (y) définie par une loi du Chi-2 de degré de liberté égal au nombre d'axes de mesure pris en compte du capteur de mouvement (CM) ;

lesdits moyens d'association (ASS) étant adaptés pour utiliser les probabilités desdites basses et hautes fréquences (BF, HF).

4. Système selon la revendication 3, dans lequel, la densité de probabilité ($P(x(n),y(n))$) d'obtention d'un couple de valeurs (($x(n)$, $y(n)$) pour la composante basses fréquences (BF) et la composante hautes fréquences (HF) étant égale au produit de la densité de probabilité ($P_{BF}(x)$) d'obtention de la valeur ($x(n)$) pour la composante basses fréquences (BF) et de la densité de probabilité ($P_{HF}(x)$) d'obtention de la valeur ($y(n)$) pour la composante hautes fréquences (HF), lesdites densités de probabilité ($P_{BF}(x)$, $P_{HF}(x)$) étant définies par les expressions suivantes :

$$\begin{cases} P_{BF}^{(p)}(x(n)) = \dfrac{1}{\sqrt{2\pi}\,\sigma_x^{(p)}} \cdot e^{-\frac{x(n)^2}{2\sigma_x^{(p)2}}} \\[3em] P_{HF}^{(p)}(y(n)) = \dfrac{1}{\sqrt{2^k}\,\sigma_y^{(p)k}\,\Gamma\left(\dfrac{k}{2}\right)}\, y(n)^{\frac{k}{2}-1}\, e^{-\frac{y(n)}{2\sigma_y^{(p)2}}} \end{cases}$$

dans lesquelles :

- k représente le degré de liberté de la composante hautes fréquences (HF) égal au nombre d'axes de mesures pris en compte dudit capteur de mouvement (CM) ;

- $\sigma_x^{(p)}$ représente la variance de x, représentative d'un type de mouvement p ;

- $\sigma_y^{(p)}$ représente la moyenne des racines carrées des énergies des composantes hautes-fréquences des axes de mesure considérés, représentative d'un type de mouvement p ;

- n représente l'indice d'échantillon ; et

- $\Gamma$ est la fonction gamma vérifiant $\Gamma\left(\dfrac{1}{2}\right) = \sqrt{\pi}$ , $\Gamma(1)=1$ et $\Gamma(z + 1) = z\,\Gamma(z)$ pour z réel.

5. Système selon l'une des revendications 2 à 4, dans lequel ledit modèle de Markov caché comprend au plus cinq états parmi une activité de repos (état 1), une activité de petite agitation (état 2), une activité de tremblements (état 3), une activité d'agitation (état 4), et une activité de forte agitation (état 5).

6. Système selon l'une des revendications 2 à 5, dans lequel les probabilités des états desdits diagrammes états-transitions sont définies par la relation suivante :

$$b_i\Big(O(n) = [x(n),\, y(n)]^T\Big) = \sum_p \alpha_{i,p} \frac{1}{\sqrt{2\pi}\,\sigma_x^{(p)}} \cdot e^{-\frac{x(n)^2}{2\sigma_x^{(p)2}}} \times \frac{1}{\sqrt{2^k}\,\sigma_y^{(p)k}\,\Gamma\left(\dfrac{k}{2}\right)} y(n)^{\frac{k}{2}-1} e^{-\frac{y(n)}{2\sigma_y^{(p)2}}}$$

dans laquelle la valeur d'un couple $\left(\sigma_x^{(p)}, \sigma_y^{(p)}\right)$ dépend de la description d'un mouvement, et les coefficients $\alpha_{i,p}$ vérifient la contrainte suivante :

$$\sum_p \alpha_{i,p} = 1 \, .$$

7. Système selon l'une des revendications précédentes, dans lequel lesdits deuxièmes moyens de détermination sont adaptés pour déterminer une deuxième probabilité d'un deuxième diagramme états-transitions de crise d'épilepsie avec des manifestations cloniques, et d'un deuxième diagramme états-transitions de crise d'épilepsie avec des manifestations toniques.

8. Système selon l'une des revendications précédentes, dans lequel les états et les probabilités des états sont identiques pour le premier diagramme états-transitions et pour les deuxièmes diagrammes états-transitions.

9. Système selon l'une des revendications 6 à 8, dans lequel lesdits de couples $\left(\sigma_x^{(i)}, \sigma_y^{(i)}\right)$ sont au nombre de 18, et obtenus par combinaison des valeurs suivantes : $\sigma_x[0]=5.10^{-3}, \sigma_x[1]=1,8.10^{-2}$, $\sigma_x[2]=3,5.10^{-2}, \sigma_x[3]=5.,510^{-2}$, $\sigma_x[4]=8.10^{-2}, \sigma_x[5]=1.10^{-1}$, et $\sigma_y[0]=1.10^{-2}, \sigma_y[1]=3.10^{-2}, \sigma_y[2]=8.10^{-2}$.

10. Système selon la revendication 9, dans lequel lesdits coefficients $\alpha_{i,p}$ sont définis comme suit, pour un couple $\left(\sigma_x^{(p)}[m], \sigma_y^{(p)}[n]\right)$, p étant un index tel que $p = m + 6n$, variant entre 0 et 17 pour les 18 couples $\left(\sigma_x^{(p)}, \sigma_y^{(p)}\right)$ :

| $\alpha_{i,p}$ | $i=1$ (repos) | $i=2$ (petite agitation) | $i=3$ (tremblements) | $i=4$ (agitation) | $i=5$ (forte agitation) |
|---|---|---|---|---|---|
| $p=0$ | 0.2564 | 0 | 0 | 0 | 0 |
| 1 | 0.0513 | 0.0526 | 0 | 0 | 0 |
| 2 | 0.02564 | 0 | 0.04 | 0 | 0 |

(suite)

| $\alpha_{i,p}$ | $i = 1$ (repos) | $i = 2$ (petite agitation) | $i = 3$ (tremblements) | $i = 4$ (agitation) | $i = 5$ (forte agitation) |
|---|---|---|---|---|---|
| 3 | 0.2564 | 0.1579 | 0.04 | 0 | 0 |
| 4 | 0.0513 | 0.2632 | 0.16 | 0 | 0 |
| 5 | 0 | 0.0526 | 0.20 | 0 | 0 |
| 6 | 0.2564 | 0.1579 | 0.04 | 0 | 0 |
| 7 | 0.0513 | 0.2632 | 0.16 | 0.0926 | 0 |
| 8 | 0 | 0.0526 | 0.20 | 0.0926 | 0 |
| 9 | 0.0256 | 0 | 0 | 0.0370 | 0 |
| 10 | 0 | 0 | 0 | 0.1852 | 0 |
| 11 | 0 | 0 | 0.16 | 0.1852 | 0 |
| 12 | 0.0256 | 0 | 0 | 0.037 | 0.0556 |
| 13 | 0 | 0 | 0 | 0.1852 | 0.0556 |
| 14 | 0 | 0 | 0 | 0.1852 | 0.0556 |
| 15 | 0 | 0 | 0 | 0 | 0.2778 |
| 16 | 0 | 0 | 0 | 0 | 0.2778 |
| 17 | 0 | 0 | 0 | 0 | 0.2778 |

**11.** Système selon la revendication 10, dans lequel un premier diagramme états-transitions des activités nocturnes générales est défini par :

| $a_{i,j}^{(1)}$ | $i = 1$ | $i = 2$ | $i = 3$ | $i = 4$ | $i = 5$ |
|---|---|---|---|---|---|
| $j = 1$ | 0.9 | 0.025 | 0.025 | 0.025 | 0.025 |
| $j = 2$ | 0.025 | 0.9 | 0.025 | 0.025 | 0.025 |
| $j = 3$ | 0.025 | 0.025 | 0.9 | 0.025 | 0.025 |
| $j = 4$ | 0.025 | 0.025 | 0.025 | 0.9 | 0.025 |
| $j = 5$ | 0.025 | 0.025 | 0.025 | 0.025 | 0.9 |

**12.** Système selon la revendication 10 ou 11, dans lequel un deuxième diagramme états-transitions de crise d'épilepsie avec des manifestations cloniques est défini par :

| $a_{i,j}^{(2)}$ | $i = 1$ | $i = 2$ | $i = 3$ | $i = 4$ | $i = 5$ |
|---|---|---|---|---|---|
| $j = 1$ | 0 | 0.3 | 0.7 | 0 | 0 |
| $j = 2$ | 0 | 0.9 | 0.1 | 0 | 0 |
| $j = 3$ | 0 | 0.1 | 0.9 | 0 | 0 |
| $j = 4$ | 0 | 0.3 | 0.7 | 0 | 0 |
| $j = 5$ | 0 | 0.3 | 0.7 | 0 | 0 |

**13.** Système selon l'une des revendications 10 à 12, dans lequel un deuxième diagramme états-transitions de crise d'épilepsie avec des manifestations toniques est défini par :

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0.7 | 0 | 0.3 |
| $j = 2$ | 0 | 0 | 0.7 | 0 | 0.3 |
| $j = 3$ | 0 | 0 | 0.9 | 0 | 0.1 |
| $j = 4$ | 0 | 0 | 0.7 | 0 | 0.3 |
| $j = 5$ | 0 | 0 | 0.3 | 0 | 0.7 |

14. Système selon l'une des revendications précédentes, comprenant, en outre, des moyens d'alerte (AL) adaptés pour prévenir d'une détection d'une crise d'épilepsie.

15. Système selon l'une des revendications précédentes, dans lequel ledit capteur de mouvement (CM) comprend un accéléromètre, un magnétomètre, ou un gyromètre.

**Patentansprüche**

1. System zum Erkennen eines epileptischen Anfalls einer ausgestreckten Person, **dadurch gekennzeichnet, dass** es Folgendes umfasst:

   - wenigstens einen Bewegungssensor (CM) mit wenigstens einer Messachse, ausgestattet mit Befestigungsmitteln (MF), um den Bewegungssensor (CM) mit der Person zu verbinden;
   - erste Mittel (DET1) zum Bestimmen einer ersten Wahrscheinlichkeit von wenigstens einem ersten Zustandsübergangsdiagramm der nächtlichen Aktivität einer ausgestreckten Person mit Bezug auf Informationen, die Messsignale des Bewegungssensors (CM) repräsentieren, wobei das erste Diagramm vordefinierte Wahrscheinlichkeiten von Übergängen umfasst, die zwischen zwei unterschiedlichen oder identischen orientierten Zuständen sind, wobei die Wahrscheinlichkeiten von Zuständen des ersten Diagramms vordefiniert sind;
   - zweite Mittel (DET2) zum Bestimmen einer zweiten Wahrscheinlichkeit von wenigstens einem zweiten Zustandsübergangsdiagramm eines epileptischen Anfalls mit Bezug auf Informationen, die Messsignale des Bewegungssensors (CM) repräsentieren, wobei ein zweites Diagramm vordefinierte Wahrscheinlichkeiten von Übergängen umfasst, die zwischen zwei unterschiedlichen oder identischen orientierten Zuständen sind, wobei die Wahrscheinlichkeiten der Zustände des zweiten Diagramms vordefiniert sind;
   - Mittel (ASS) zum Assoziieren eines Zustands der Person in Abhängigkeit von Wahrscheinlichkeiten von Messsignalen des Bewegungssensors (CM);
   - erste Mittel (CALC1) zum Berechnen Verhältnissen $(\varphi_1, \varphi_1)$ der ersten Wahrscheinlichkeit und der zweiten Wahrscheinlichkeit; und
   - Mittel (DETEC) zum Erkennen eines epileptischen Anfalls, wenn wenigstens eines der berechneten Verhältnisse $(\varphi_1, \varphi_1)$ kleiner ist als eine Schwelle $(\lambda_1, \lambda_2)$.

2. System nach Anspruch 1, bei dem wenigstens eines der Zustandsübergangsdiagramme zum Nutzen eines verdeckten Markov-Modells ausgelegt ist.

3. System nach Anspruch 2, das ferner Folgendes umfasst:

   - ein Filter (FILT) zum Wählen, für jede Messachse des Bewegungssensors (CM), von hohen Frequenzen (HF), die höher sind als eine erste Schwelle (S1), und von tiefen Frequenzen (BF), die tiefer sind als eine zweite Schwelle (S2), die gleich oder kleiner ist als die erste Schwelle (S1);
   - zweite Mittel (CALC2) zum Berechnen eines ersten Wertes (x) gleich einer linearen Kombination von jeweiligen Variationen zwischen zwei aufeinander folgenden Zeitintervallen, gemäß jeder Messachse, der tiefen Frequenzen (BF) pro Zeitintervall (n);
   - dritte Mittel (CALC3) zum Berechnen eines zweites Wertes (y) gleich dem Mittel von Energien, gemäß jeder Messachse, der hohen Frequenzen (HF);
- dritte Mittel (DET3) zum Bestimmen der Wahrscheinlichkeit ($P_{BF}(x)$) des ersten Wertes (x), definiert durch ein normales zentriertes Gaußsches Gesetz; und
- vierte Mittel (DET4) zum Bestimmen der Wahrscheinlichkeit ($P_{HF}(x)$) des zweiten Wertes (y), definiert durch ein Chi-2 Freiheitsgradegesetz gleich der Anzahl von Messachsen, die von dem Bewegungssensor (CM) berücksichtigt werden;

wobei die Assoziationsmittel (ASS) zum Nutzen der Wahrscheinlichkeiten der tiefen und hohen Frequenzen (BF, HF) adaptiert sind.

4. System nach Anspruch 3, bei dem die Wahrscheinlichkeitsdichte ($P(x(n),y(n))$) des Erhaltens eines Paares von Werten ($(x(n), y(n))$) für die Niederfrequenzkomponente (BF) und die Hochfrequenzkomponente (HF) gleich dem Produkt der Wahrscheinlichkeitsdichte ($P_{BF}(x)$) des Erhaltens des Wertes ($x(n)$) für die Niederfrequenzkomponente (BF) und die Wahrscheinlichkeitsdichte ($P_{HF}(x)$) des Erhaltens des Wertes ($y(n)$) für die Hochfrequenzkomponente (HF) ist, wobei die Wahrscheinlichkeitsdichten ($P_{BF}(x)$, $P_{HF}(x)$) durch die folgenden Ausdrücke definiert werden:

$$\begin{cases} P_{BF}^{(p)}\left(x(n)\right) = \dfrac{1}{\sqrt{2\pi}\,\sigma_x^{(p)}} \cdot e^{-\frac{x(n)^2}{2\sigma_x^{(p)2}}} \\[4mm] P_{HF}^{(p)}\left(y(n)\right) = \dfrac{1}{\sqrt{2^k}\,\sigma_y^{(p)k}\,\Gamma\!\left(\frac{k}{2}\right)}\, y(n)^{\frac{k}{2}-1} e^{-\frac{y(n)}{2\sigma_y^{(p)2}}} \end{cases}$$

wobei:

- k den Freiheitsgrad der Hochfrequenzkomponente (HF) repräsentiert und der Anzahl von berücksichtigten Messachsen von dem Bewegungssensor (CM)gleicht,

- $\sigma_x^{(p)}$ die Varianz von x repräsentiert und die für einen Bewegungstyp p repräsentativ ist;

- $\sigma_y^{(p)}$ das Mittel der Quadratwurzeln von Energien von Hochfrequenzkomponenten der betrachteten Messachsen repräsentiert und für einen Bewegungstyp p repräsentativ ist;
- n den Abtastindex repräsentiert; und

- $\Gamma$ die Gamma-Funktion ist, die $\Gamma\!\left(\frac{1}{2}\right) = \sqrt{\pi}$, $\Gamma(1) = 1$ und $\Gamma(z + 1) = z\,\Gamma(z)$ für z reell verifiziert.

5. System nach einem der Ansprüche 2 bis 4, wobei das verdeckte Markov-Modell höchstens fünf Zustände aus einer Ruheaktivität (Zustand 1), einer Aktivität mit geringer Agitation (Zustand 2), einer Zitteraktivität (Zustand 3), einer Agitationsaktivität (Zustand 4) und einer Aktivität mit starker Agitation (Zustand 5) umfasst.

6. System nach einem der Ansprüche 2 bis 5, bei dem die Wahrscheinlichkeiten der Zustände der Zustandsübergangsdiagramme durch die folgende Relation definiert werden:

$$b_i\left(O(n) = [x(n), y(n)]^T\right) = \sum_p \alpha_{i,p}\, \frac{1}{\sqrt{2\pi}\,\sigma_x^{(p)}} \cdot e^{-\frac{x(n)^2}{2\sigma_x^{(p)2}}} \times \frac{1}{\sqrt{2^k}\,\sigma_y^{(p)k}\,\Gamma\!\left(\frac{k}{2}\right)}\, y(n)^{\frac{k}{2}-1} e^{-\frac{y(n)}{2\sigma_y^{(p)2}}}$$

wobei der Wert eines Paares $\left(\sigma_x^{(p)}, \sigma_y^{(p)}\right)$ von der Beschreibung einer Bewegung abhängt und die Koeffizienten $\alpha_{i,p}$ die folgende Beschränkung verifizieren: $\sum_p \alpha_{i,p} = 1$.

7. System nach einem der vorherigen Ansprüche, bei dem die zweiten Bestimmungsmittel zum Bestimmen einer zweiten Wahrscheinlichkeit eines zweiten Zustandsübergangsdiagramms eines epileptischen Anfalls mit klonischen Manifestationen und eines zweiten Zustandsübergangsdiagramms eines epileptischen Anfalls mit tonischen Manifestationen ausgelegt sind.

8. System nach einem der vorherigen Ansprüche, bei dem die Zustände und die Wahrscheinlichkeiten der Zustände für das erste Zustandsübergangsdiagramm und für die zweiten Zustandsübergangsdiagramme identisch sind.

9. System nach einem der Ansprüche 6 bis 8, bei dem die Zahl der Paare $\left(\sigma_x^{(i)}, \sigma_y^{(i)}\right)$ 18 beträgt und durch Kombinieren der folgenden Werte erhalten wird: $\sigma_x[0]=5\times10^{-3}$, $\sigma_x[1]=1,8\times10^{-2}$, $\sigma_x[2]=3,5\times10^{-2}$, $\sigma_x[3]=5,5\times10^{-2}$, $\sigma_x[4]=8\times10^{-2}$, $\sigma_x[5]=1\times10^{-1}$, and $\sigma_y[0]=1\times10^{-2}$, $\sigma_y[1]=3\times10^{-2}$, $\sigma_y[2]=8\times10^{-2}$.

10. System nach Anspruch 9, bei dem die Koeffizienten $\alpha_{ip}$ wie folgt für ein Paar $\left(\sigma_x^{(p)}[m], \sigma_y^{(p)}[n]\right)$ definiert werden, wobei p ein Index *wie* $p = m + 6n$ ist, der zwischen 0 und 17 für die 18 Paare $\left(\sigma_x^{(p)}, \sigma_y^{(p)}\right)$ variiert:

| $\alpha_{i,p}$ | $i = 1$ (Ruhe) | $i = 2$ (geringe Agitation) | $i = 3$ (Zittern) | $i = 4$ (Agitation) | $i = 5$ (starke Agitation) |
|---|---|---|---|---|---|
| $p = 0$ | 0,2564 | 0 | 0 | 0 | 0 |
| 1 | 0,0513 | 0,0526 | 0 | 0 | 0 |
| 2 | 0,02564 | 0 | 0,04 | 0 | 0 |
| 3 | 0,2564 | 0,1579 | 0,04 | 0 | 0 |
| 4 | 0,0513 | 0,2632 | 0,16 | 0 | 0 |
| 5 | 0 | 0,0526 | 0,20 | 0 | 0 |
| 6 | 0,2564 | 0,1579 | 0,04 | 0 | 0 |
| 7 | 0,0513 | 0,2632 | 0,16 | 0,0926 | 0 |
| 8 | 0 | 0,0526 | 0,20 | 0,0926 | 0 |
| 9 | 0,0256 | 0 | 0 | 0,0370 | 0 |
| 10 | 0 | 0 | 0 | 0,1852 | 0 |
| 11 | 0 | 0 | 0,16 | 0,1852 | 0 |
| 12 | 0,0256 | 0 | 0 | 0,037 | 0,0556 |
| 13 | 0 | 0 | 0 | 0,1852 | 0,0556 |
| 14 | 0 | 0 | 0 | 0,1852 | 0,0556 |
| 15 | 0 | 0 | 0 | 0 | 0,2778 |
| 16 | 0 | 0 | 0 | 0 | 0,2778 |
| 17 | 0 | 0 | 0 | 0 | 0,2778 |

11. System nach Anspruch 10, bei dem ein erstes Zustandsübergangsdiagramm von allgemeinen nächtlichen Aktivitäten wie folgt definiert wird:

| $a_{i,j}^{(1)}$ | $i = 1$ | $i = 2$ | $i = 3$ | $i = 4$ | $i = 5$ |
|---|---|---|---|---|---|
| $j = 1$ | 0,9 | 0,025 | 0,025 | 0,025 | 0,025 |
| $j = 2$ | 0,025 | 0,9 | 0,025 | 0,025 | 0,025 |
| $j = 3$ | 0,025 | 0,025 | 0,9 | 0,025 | 0,025 |
| $j = 4$ | 0,025 | 0,025 | 0,025 | 0,9 | 0,025 |

(fortgesetzt)

| $a_{i,j}^{(1)}$ | $i = 1$ | $i = 2$ | $i = 3$ | $i = 4$ | $i = 5$ |
|---|---|---|---|---|---|
| $j = 5$ | 0,025 | 0,025 | 0,025 | 0,025 | 0,9 |

**12.** System nach Anspruch 10 oder 11, bei dem ein zweites Zustandsübergangsdiagramm eines epileptischen Anfalls mit klonischen Manifestationen definiert wird durch:

| $a_{i,j}^{(2)}$ | $i = 1$ | $i = 2$ | $i = 3$ | $i = 4$ | $i = 5$ |
|---|---|---|---|---|---|
| $j = 1$ | 0 | 0,3 | 0,7 | 0 | 0 |
| $j = 2$ | 0 | 0,9 | 0,1 | 0 | 0 |
| $j = 3$ | 0 | 0,1 | 0,9 | 0 | 0 |
| $j = 4$ | 0 | 0,3 | 0,7 | 0 | 0 |
| $j = 5$ | 0 | 0,3 | 0,7 | 0 | 0 |

**13.** System nach einem der Ansprüche 10 bis 12, bei dem ein zweites Zustandsübergangsdiagramm für einen epileptischen Anfall mit tonischen Manifestationen definiert wird durch:

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0,7 | 0 | 0,3 |
| $j = 2$ | 0 | 0 | 0,7 | 0 | 0,3 |
| $j = 3$ | 0 | 0 | 0,9 | 0 | 0,1 |
| $j = 4$ | 0 | 0 | 0,7 | 0 | 0,3 |
| $j = 5$ | 0 | 0 | 0,3 | 0 | 0,7 |

**14.** System nach einem der vorherigen Ansprüche, das ferner Alarmierungsmittel (AL) umfasst, ausgelegt zum Melden einer Erkennung eines epileptischen Anfalls.

**15.** System nach einem der vorherigen Ansprüche, bei dem der Bewegungssensor (CM) einen Beschleunigungsmesser, einen Magnetometer oder einen Wendekreisel umfasst.

**Claims**

**1.** A system for detecting an epileptic seizure in a lying person, **characterised in that** it comprises:

- at least one motion sensor (CM) with at least one measurement axis provided with fastening elements (MF) for attaching said motion sensor (CM) to said person;
- first determination means (DET1) for determining a first probability of at least a first state transition diagram of the nocturnal activity of a lying person with respect to data representing the measurement signals of the motion sensor (CM), said first diagram comprising predetermined probabilities of oriented transitions between two different or identical states, the probabilities of the states of said first diagram being predetermined;
- second determination means (DET2) for determining a second probability of at least a second state transition diagram for an epileptic seizure with respect to data representing the measurement signals of the motion sensor (CM), a second diagram comprising predetermined probabilities of oriented transitions between two different or identical states, the probabilities of the states of said second diagram being predetermined;
- association means (ASS) for associating a state of said person as a function of the probabilities of the measurement signals of the motion sensor (CM);
- first calculation means (CALC1) for calculating ratios ($\varphi_1$, $\varphi_1$) between the first probability and the second probability; and
- detection means (DETEC) for detecting an epileptic seizure when at least one of said calculated relations ($\varphi_1$, $\varphi_1$) is below a threshold ($\lambda_1$, $\lambda_2$).

2. The system according to Claim 1, wherein at least one of said state transition diagrams is adapted to use a hidden Markov model.

3. The system according to Claim 2, further comprising:

   - a filter (FILT) for selecting, for each measurement axis of the motion sensor (CM), high frequencies (HF) above a first threshold (S1) and low frequencies (BF) below a second threshold (S2) which is lower than or equal to said first threshold (S1);
   - second calculation means (CALC2) for calculating a first value (x) equal to a linear combination of the respective variations, along each measurement axis, between two successive time intervals of said low frequencies (BF) per time interval (n);
   - third calculation means (CALC3) for calculating a second value (y) equal to the mean of the energies, along each measurement axis, of said high frequencies (HF);
   - third determination means (DET3) for determining the probability ($P_{BF}(x)$) of said first value (x) defined by a normal centred Gaussian distribution; and
   - fourth determination means (DET4) for determining the probability ($P_{HF}(x)$) of said second value (y) defined by a Chi-2 distribution with a degree of freedom equal to the number of measurement axes of the motion sensor (CM) taken into consideration;

   said association means (ASS) being adapted to use the probabilities of said low and high frequencies (BF, HF).

4. The system according to Claim 3, wherein the probability density (P(x(n),y(n))) of obtaining a pair of values ((x(n), y(n)) for the low frequency component (BF) and the high frequency component (HF) being equal to the product of the probability density ($P_{BF}(x)$) of obtaining the value (x(n)) for the low frequency component (BF) and the probability density ($P_{BF}(x)$) of obtaining the value (y(n)) for the high frequency component (HF), said probability densities ($P_{BF}(x)$, $P_{BF}(x)$) being defined by the following expressions:

$$\begin{cases} P_{BF}^{(p)}(x(n)) = \dfrac{1}{\sqrt{2\pi}\,\sigma_x^{(p)}} \cdot e^{-\frac{x(n)^2}{2\sigma_x^{(p)2}}} \\[4ex] P_{HF}^{(p)}(y(n)) = \dfrac{1}{\sqrt{2^k}\,\sigma_y^{(p)k}\,\Gamma\left(\dfrac{k}{2}\right)} y(n)^{\frac{k}{2}-1} e^{-\frac{y(n)}{2\sigma_y^{(p)2}}} \end{cases}$$

wherein:

   - k represents the degree of freedom of the high frequency component (HF) and equals the number of measurement axes of said motion sensor (CM) taken into consideration;

   - $\sigma_x^{(p)}$ represents the variance of x and is representative of a type of movement p;

   - $\sigma_y^{(p)}$ represents the mean of the square roots of the energies of the high frequency components of the measurement axes considered and is representative of a type of movement p;
   - n represents the sample index: and

   - $\Gamma$ is the gamma function obeying $\Gamma\left(\dfrac{1}{2}\right) = \sqrt{\pi}$, $\Gamma(1) = 1$ and $\Gamma(z + 1) = z\Gamma(z)$ where z is real.

5. The system according to any of Claims 2 to 4, wherein said hidden Markov model comprises no more than five states chosen from a group comprising a rest activity (state 1), a slight agitation activity (state 2), a tremor activity (state 3), an agitation activity (state 4) and a strong agitation activity (state 5).

6. The system according to any of Claims 2 to 5, wherein the probabilities of the states of said state transition diagrams are defined by the following relation:

$$b_i\left(O(n) = [x(n), y(n)]^T\right) = \sum_p \alpha_{i,p} \frac{1}{\sqrt{2\pi}\,\sigma_x^{(p)}} \cdot e^{-\frac{x(n)^2}{2\sigma_x^{(p)2}}} \times \frac{1}{\sqrt{2^k}\,\sigma_y^{(p)k}\,\Gamma\left(\frac{k}{2}\right)} y(n)^{\frac{k}{2}-1} e^{-\frac{y(n)}{2\sigma_y^{(p)2}}}$$

wherein the value of a pair $\left(\sigma_x^{(p)}, \sigma_y^{(p)}\right)$ depends on the description of a movement, and the coefficients $\alpha_{i,p}$ comply with the following constraint:

$$\sum_p \alpha_{i,p} = 1.$$

7. The system according to any of the preceding claims, wherein said second determination means are adapted to determine a second probability of a second state transition diagram for an epileptic seizure with clonic manifestations and of a second state transition diagram for an epileptic seizure with tonic manifestations.

8. The system according to any of the preceding claims, wherein the states and the probabilities of the states are identical for the first state transition diagram and for the second state transition diagrams.

9. The system according to any of Claims 6 to 8, wherein there are 18 of said pairs $\left(\sigma_x^{(i)}, \sigma_y^{(i)}\right)$, which are obtained by the combination of the following values:

$$\sigma_x[0] = 5\times10^{-3}, \ \sigma_x[1] = 1.8\times10^{-2}, \ \sigma_x[2] = 3.5\times10^{-2}, \ \sigma_x[3] = 5.5\times10^{-2}, \ \sigma_x[4] = 8\times10^{-2}, \ \sigma_x[5] = 1\times10^{-1}, \text{ and } \sigma_y[0] = 1\times10^{-2}, \ \sigma_y[1] = 3\times10^{-2}, \ \sigma_y[2] = 8\times10^{-2}.$$

10. The system according to Claim 9, wherein said coefficients $\alpha_{i,p}$ are defined as follows, for a pair $\left(\sigma_x^{(p)}[m], \sigma_y^{(p)}[n]\right)$, where p is an index such as $p = m+6n$, varying from 0 to 17 for the 18 pairs $\left(\sigma_x^{(p)}, \sigma_y^{(p)}\right)$:

| $\alpha_{i,p}$ | $i = 1$ (rest) | $i = 2$ (slight agitation) | $i = 3$ (tremors) | $i = 4$ (agitation) | $i = 5$ (strong agitation) |
|---|---|---|---|---|---|
| $p = 0$ | 0.2564 | 0 | 0 | 0 | 0 |
| 1 | 0.0513 | 0.0526 | 0 | 0 | 0 |
| 2 | 0.02564 | 0 | 0.04 | 0 | 0 |
| 3 | 0.2564 | 0.1579 | 0.04 | 0 | 0 |
| 4 | 0.0513 | 0.2632 | 0.16 | 0 | 0 |
| 5 | 0 | 0.0526 | 0.20 | 0 | 0 |
| 6 | 0.2564 | 0.1579 | 0.04 | 0 | 0 |
| 7 | 0.0513 | 0.2632 | 0.16 | 0.0926 | 0 |
| 8 | 0 | 0.0526 | 0.20 | 0.0926 | 0 |

(continued)

| $\alpha_{i,p}$ | $i = 1$ (rest) | $i = 2$ (slight agitation) | $i = 3$ (tremors) | $i = 4$ (agitation) | $i = 5$ (strong agitation) |
|---|---|---|---|---|---|
| 9 | 0.0256 | 0 | 0 | 0.0370 | 0 |
| 10 | 0 | 0 | 0 | 0.1852 | 0 |
| 11 | 0 | 0 | 0.16 | 0.1852 | 0 |
| 12 | 0.0256 | 0 | 0 | 0.037 | 0.0556 |
| 13 | 0 | 0 | 0 | 0.1852 | 0.0556 |
| 14 | 0 | 0 | 0 | 0.1852 | 0.0556 |
| 15 | 0 | 0 | 0 | 0 | 0.2778 |
| 16 | 0 | 0 | 0 | 0 | 0.2778 |
| 17 | 0 | 0 | 0 | 0 | 0.2778 |

**11.** The system according to Claim 10, wherein a first state transition diagram of general nocturnal activity is defined by:

| $a_{i,j}^{(1)}$ | $i = 1$ | $i = 2$ | $i = 3$ | $i = 4$ | $i = 5$ |
|---|---|---|---|---|---|
| $j = 1$ | 0.9 | 0.025 | 0.025 | 0.025 | 0.025 |
| $j = 2$ | 0.025 | 0.9 | 0.025 | 0.025 | 0.025 |
| $j = 3$ | 0.025 | 0.025 | 0.9 | 0.025 | 0.025 |
| $j = 4$ | 0.025 | 0.025 | 0.025 | 0.9 | 0.025 |
| $j = 5$ | 0.025 | 0.025 | 0.025 | 0.025 | 0.9 |

**12.** The system according to Claim 10 or 11, wherein a second state transition diagram for an epileptic seizure with clonic manifestations is defined by:

| $a_{i,j}^{(2)}$ | $i = 1$ | $i = 2$ | $i = 3$ | $i = 4$ | $i = 5$ |
|---|---|---|---|---|---|
| $j = 1$ | 0 | 0.3 | 0.7 | 0 | 0 |
| $j = 2$ | 0 | 0.9 | 0.1 | 0 | 0 |
| $j = 3$ | 0 | 0.1 | 0.9 | 0 | 0 |
| $j = 4$ | 0 | 0.3 | 0.7 | 0 | 0 |
| $j = 5$ | 0 | 0.3 | 0.7 | 0 | 0 |

**13.** The system according to any of Claims 10 to 12, wherein a second state transition diagram for an epileptic seizure with tonic manifestations is defined by:

| $a_{i,j}^{(3)}$ | $i = 1$ | $i = 2$ | $i = 3$ | $i = 4$ | $i = 5$ |
|---|---|---|---|---|---|
| $j = 1$ | 0 | 0 | 0.7 | 0 | 0.3 |
| $j = 2$ | 0 | 0 | 0.7 | 0 | 0.3 |
| $j = 3$ | 0 | 0 | 0.9 | 0 | 0.1 |

(continued)

| $a_{i,j}^{(3)}$ | $i = 1$ | $i = 2$ | $i = 3$ | $i = 4$ | $i = 5$ |
|---|---|---|---|---|---|
| $j = 4$ | 0 | 0 | 0.7 | 0 | 0.3 |
| $j = 5$ | 0 | 0 | 0.3 | 0 | 0.7 |

14. The system according to any of the preceding claims, further comprising alerting means (AL) adapted for providing a warning of the detection of an epileptic seizure.

15. The system according to any of the preceding claims, wherein said motion sensor (CM) comprises an accelerometer, a magnetometer or a gyrometer.

MF

BT

CM
FILT
ASS
CALC1
DET1
DET2
DETEC
CALC2
CALC3
DET3
DET4

AL

FIG.1

CM

BT

AL
FILT
ASS
CALC1
DET1
DET2
DETEC
CALC2
CALC3
DET3
DET4
OP

FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2009062696 A
- US 6212510 B

**Littérature non-brevet citée dans la description**

- **DE S. BONNET ; R. HELIOT.** A magnetometer-based approach for studying human movements. *IEEE Transactions on Biomedical Engineering,* 2007, vol. 54 (7
- **A. CHWALEBA ; J. JAKUBOWSKI ; K. KWIATOS.** The measuring set and signal processing method for the characterization of human hand tremor. *CADSM,* 2003
- **J. D. FROST.** Triaxial accelerometry: a method for quantifying tremor and ataxia. *IEEE Transactions on Biomedical Engineering,* 1978, vol. 25 (49
- **T. NIJSEN.** The potential value of 3d accelerometry for detection of motor seizures in severe epilepsy. *Epilepsy and Behavior,* 2005, vol. 7
- **T. NIJSEN.** Detection of subtle nocturnal motor activity from 3d accelerometry recordings in epilepsy patients. *IEEE Transactions on Biomedical Engineering,* 2007, vol. 54
- **JIN HE et al.** Real-time Daily Activity Classification with Wireless Sensor Networks using Hidden Markov Model. *IEEE Engineering in medicine and biology society,* 2007, 3192-3195
- **L.R. RABINER ; B.H. JUANG.** An introduction to hidden Markov models. *IEEE ASSP Magazine,* Janvier 1986
- **CAPPÉ ; MOULINES ; RYDEN.** Inference in Hidden Markov Models. Springer